# EUROPEAN PATENT SPECIFICATION

(11) **EP 4 652 460 B1**
(45) Date of publication and mention of the grant of the patent: **27.05.2026**
(21) Application number: 24712343.3
(22) Date of filing: 15.01.2024
(51) Int. Cl.: G01N 33/68, G01N 33/66

(54) **A METHOD OF DETECTING THE CONGENITAL DISORDER OF GLYCOSYLATION USING THE PANEL OF LECTINS**
VERFAHREN ZUM NACHWEIS VON ANGEBORENER GLYCOSYLIERUNGSERKRANKUNG UNTER VERWENDUNG VON LEKTINEN
PROCÉDÉ DE DÉTECTION D'UN TROUBLE CONGÉNITAL DE GLYCOSYLATION À L'AIDE D'UN PANEL DE LECTINES

(30) Priority: 17.01.2023 SK 500022023
(43) Date of publication of application: 26.11.2025
(73) Proprietor: CHEMICKY USTAV SLOVENSKEJ AKADEMIE VIED, VEREJNA VYSKUMNA INSTITUCIA, 845 38 Bratislava - mestska cast Karlova Ves (SK)
(72) Inventor: KATRLIK, Jaroslav, 831 03 Bratislava (SK); PAKANOVA, Zuzana, 811 06 Bratislava (SK)
(74) Representative: Majlingová, Zuzana
(86) International application number: PCT/SK2024/050001
(87) International publication number: WO 2024/155244

(56) References cited:
- KATRLÍK JAROSLAV ET AL: "Application of lectin-based protein microarray in glyco-biomarker analysis of congenital disorder of glycosylation", 24TH INTERNATIONAL SYMPOSIUM ON GLYCOCONJUGATES, vol. 34, 27 August 2017 (2017-08-27), pages S60 - S61, XP093148347, Retrieved from the Internet <URL:https://link.springer.com/content/pdf/10.1007/s10719-017-9784-5.pdf>
- VAN DIJK^A W ET AL: "Increased @a3-fucosylation of @a"1-acid glycoprotein in patients with congenital disorder of glycosylation type IA (CDG-Ia)^1", FEBS LETTERS, ELSEVIER, AMSTERDAM, NL, vol. 494, no. 3, 13 April 2001 (2001-04-13), pages 232 - 235, XP004235076, ISSN: 0014-5793, DOI: 10.1016/S0014-5793(01)02349-3
- DI PATRIA LAURA ET AL: "Defective IGF-1 prohormone N-glycosylation and reduced IGF-1 receptor signaling activation in congenital disorders of glycosylation", CMLS CELLULAR AND MOLECULAR LIFE SCIENCES, BIRKHAUSER VERLAG, HEIDELBERG, DE, vol. 79, no. 3, 24 February 2022 (2022-02-24), XP037701424, ISSN: 1420-682X, [retrieved on 20220224], DOI: 10.1007/S00018-022-04180-X

## Description

### Technical field

A quick biochemical determination in the way of biochips or microplates microarray using the panel of lectins in order to identify glyco-epitopes for determining the congenital disorders of glycosylation, eventually for suggesting their possible subtypes.

### Background Art

Glycosylation is a co- and post-translational modification of proteins, peptides and lipids that is important for their correct physiological function. Changes in the glyco-pattern /glyco-epitope are connected to changes in their biological function and are involved in many civilisational and metabolic diseases.

Until now the method of customary isoelectric focusing (IEF) of blood transferrin and immunodetection have been used to detect any congenital disorders of glycosylation (CDG) (Hackler, R., et al. (1995) Effect of separation conditions on automated isoelectric focusing of carbohydrate-deficient transferrin and other human isotransferrins using the PhastSystem. Anal Biochem 230:281-289), eventually mass spectrometry (MALDI/TOF-MS, ESI-MS) (van Scherpenzeel, M., et al. (2015) High-resolution mass spectrometry glycoprofiling of intact transferrin for diagnosis and subtype identification in the congenital disorders of glycosylation. Transl. Research 166(6): 639-649.e1). Though, the IEF based method is not able to determine some subtypes of congenital disorders of glycosylation as some partial glycosylation reactions, for example fucosylation, are unidentifiable by traditional methods (Park J.H., et al. (2020) L-Fucose treatment of FUT8-CDG. Mol Genet Metab Rep 5(25):100680). By the mass spectrometry of released and derivatized glyco-epitopes, it is possible to determine their fucosylation, nevertheless the method is demanding financially, time wise as well as in view of requirements on staff expertise. Therefore, the lectin panels are suitable and available means for determination of congenital disorders of glycosylation even within the frame of a routine screening.

Two patents are solving the determination of abnormal glycosylation. The first one, marked as CA02534815, covers only a small group of selected lectins. In this case, the invention is based on detection via lectins immobilized on a solid base, while the sample itself is not immobilized. The sample is not even denatured, whereas denaturation plays a significant role in steric exposure of glyco-epitopes. Neither spectrophotometric nor fluorescent detection is used for determination purposes. Determination is carried out by evanescent optical technique and by detecting light reflected from a solid base, which reduces availability and increases its financial costiveness. The second patent, marked as JP2008032520A, is based on a formation of a specific protein complex with, first, a specific antibody (like monoclonal anti-transferrin antibody) that must be deglycosylated, eventually the glycan must be protected; and then binding of this complex to a solid surface (balls) that i.) prolongs the time and financial demands, ii.) is not an essential step for determining CDG, and iii.) is not a suitable way for determining all serum glyco-epitopes at the same time.

All the above-mentioned shortcomings may be solved by the quick determination of congenital disorders of glycosylation / a screening for possible CDG presence by determining the complex glyco-epitopes of a specific biological fluid, serum or plasma, using its denaturation, immobilisation on a solid base, an application of a specific combination of biotinylated lectins, and detection using commonly used microplate readers or microarray scanners.

Katrlík J., et al. ((2017). Glycoconj J 34(Suppl 1):S60-S61) describes the application of lectin-based protein microarray in glyco-biomarker analysis of congenital disorder of glycosylation.

### Summary of Invention

The aim of the present invention is to detect a primary congenital disorder of glycosylation in a sample of human serum or blood plasma that may be denatured, based on an analysis of the total serum glyco-epitopes using the specifically designed biochips or microplates that contain immobilized samples and their detection using a specific combination of biotinylated lectins.

Based on analytical determination in the format of microarray biochips or microplates, this approach will allow determining the difference in glycosylation of sera when determining the specific primary deficit in the glycosylation pathway as well as in case of other diseases manifested by changes in glycosylation. The ratio of signals obtained from the synergistic combination of individual lectins is used according to this invention as the main distinguishing factor between CDG and non-CDG. The method includes glycosylation analysis of the sample in order to determine congenital disorders of glycosylation, while the sample is human blood serum which is diluted and may be denatured (by temperature or otherwise - for example, chemical agents, microwave radiation, eventually combination thereof). Serum denaturation represents an innovative approach, as under defined conditions it will significantly increase the reactive capacity of glyco-epitopes for their binding with lectins within the panel, while it has got no negative impact on their glycan arrangement or relative composition. Immobilization of a sample is another innovative approach. This significantly reduces demands on its quantity (which is, for example, in the case of serum from child patients an important factor). There is the option to store the prepared biochips / microplates with samples and use them for future analyses. Immobilization of a sample can be done either physically or chemically. Physical immobilization is carried out by sorption of a sample onto a surface that is covered in a layer of material capable of sorbing the proteins, such as with a layer of cellulose nitrate or a layer of a different polymer capable of binding proteins. Chemical immobilization is carried out by applying a sample to the surface that is modified by functional groups which react with the proteins to form a chemical bond, while these functional groups can be, for example, epoxides, aldehydes or N-hydroxysuccinimides. A significant advantage of this invention is the simplicity and speed in which a result is achieved, because commercially available reagents with guaranteed quality are used, especially in the case of a microplate realisation for which widely distributed reading devices can be used.

The subject matter of this invention is the method of detecting congenital disorder of glycosylation using the panel of lectins containing the following successive steps:
a) Dilution of the sample of analyzed human blood serum or blood plasma as well as the control serum or plasma with a physiological solution to a concentration within 1:100 and 1:1000000;
b) Preparing the microarray biochip or microplate through application and physical or chemical immobilisation of the applied analyzed sample and of the control serum or plasma onto the surface of the microarray substrate or into the wells of a microplate;
c) Incubation of the prepared microarray biochip or microplate with the samples with the panel of biotinylated lectins the concentration of which ranges from 0.01 up to 100 µg/ml and the subsequent incubation with a fluorescently or spectrophotometrically labelled conjugate of streptavidin or another non-glycosylated protein; where the panel of lectins is a set of minimum two lectins with the same or different dominant glycan specificity and where the panel of lectins contains lectins in the form of isolated proteins or recombinant proteins: Aleuria aurantia lectin (AAL), Arachis hypogaea agglutinin (PNA), Concanavalin A (Con A), Datura stramonium lectin (DSL), Galanthus nivalis lectin (GNL), Griffonia simplicifolia lectin-I (GSL-I), Griffonia simplicifolia lectin-II (GSL-II), Hippeastrum hybrid lectin (HHL), Lens culinaris agglutinin (LCA), Lotus tetragonolobus lectin (LTL), Maackia amurensis lectin-I (MAL-I), Maackia amurensis lectin-II (MAL-II), Narcissus pseudonarcissus (daffodil) lectin (NPL), Phaseolus vulgaris erythtroagglutinin (PHA-E), Phaseolus vulgaris leukoagglutinin (PHA-L), Ricinus communis agglutinin-I (RCA), Sambucus nigra agglutinin (SNA), Ulex europaeus agglutinin I (UEA-I), Wheat germ agglutinin (WGA), Aspergillus oryzae lectin (AOL) where there are lectins with a dominant glycan specificity for fucose (Fuc), for galactose (Gal), for sialic acid (Sia), for mannose (Man), for N-acetylgalactosamine (GalNAc), or for N-acetylglucosamine (GlcNAc);
d) In the case of using a fluorescent label, the signal detection is performed by measuring fluorescence intensity, in the case of labelling with peroxidase, the signal detection is performed spectrophotometrically, it is always performed using the appropriate reading device;
e) Determining the presence of a congenital disorder of glycosylation, or its possible subtype, by calculating the value of the ratio of the interaction signals of control serum, or plasma, with a pair of lectins from the panel, and by proportioning this value with the value of the ratio of the interaction signals of the examined sample with the same pair of lectins from the panel; while in case that the resulting ratio substantially differentiates from value of 1, is lesser than 0,5 or greater than 2, the examined sample shall be evaluated as positive for the possible presence of a congenital disorder of glycosylation, while the order in which the lectins in the pair were used does not matter.

According to the preferred embodiment, in step a) diluted blood serum or plasma, as well as control serum or plasma, shall be denatured using chemical agents and/or temperature and/or microwave radiation, or their combination, in this step.

According to another preferred embodiment, a microarray substrate or a sorption microplate is a carrier.

Based on another preferred embodiment, pairs of lectins for calculation of the ratio value of the sample interaction signals with these lectins are GNL/SNA, ConA/SNA, HHL/SNA, NPL/SNA, LCA/SNA, PHA-E/SNA, PHA-L/SNA, RCA/SNA, WGA/SNA, DSL/SNA, PNA/SNA, GSL-I/SNA, GSL-II/SNA, AAL/SNA, LTL/SNA, UEA-I/SNA, GNL/MAL-I, ConA/MAL-I, HHL/MAL-I, NPL/MAL-I, LCA/MAL-I, PHA-E/MAL-I, PHA-L/MAL-I, RCA/MAL-I, AOL/MAL-I, WGA/MAL-I, DSL/MAL-I, PNA/MAL-I, GSL-I/MAL-I, GSL-II/MAL-I, AAL/MAL-I, LTL/MAL-I, UEA-I/MAL-I, GNL/MAL-II, ConA/MAL-II, HHL/MAL-II, NPL/MAL-II, LCA/MAL-II, PHA-E/MAL-II, PHA-L/MAL-II, RCA/MAL-II, AOL/MAL-II, PHA-E/ConA, PHA-E/GNL, PHA-L/ConA, PHA-L/GNL, WGA/MAL-II, DSL/MAL-II, PNA/MAL-II, GSL-I/MAL-II, GSL-II/MAL-II, AAL/MAL-II, LTL/MAL-II, UEA-I/MAL-II, AOL/ConA, AOL/SNA, AOL/GNL, PNA/GNL, LTL/GNL, UEA-I/GNL, RCA/GNL, AAL/ConA, AAL/GNL, AAL/HHL, AAL/NPL, AOL/HHL, AOL/NPL, GSL-I/ConA, GSL-I/GNL, GSL-I/HHL, GSL-I/NPL, GSL-I/LCA, DSL/ConA, DSL/GNL, DSL/HHL, DSL/NPL, DSL/LCA, GSL-II/ConA, GSL-II/GNL, GSL-II/HHL, GSL-II/NPL, GSL-II/LCA, WGA/ConA, WGA/GNL, WGA/HHL, WGA/NPL, WGA/LCA, LTL/ConA, UEA-I/ConA, PNA/ConA, RCA/ConA, GNL/ConA.

According to another preferred embodiment, pairs of lectins to calculate the ratio value of the sample interaction signals with these lectins are GNL/SNA, ConA/SNA, HHL/SNA, NPL/SNA, LCA/SNA, PHA-E/SNA, PHA-L/SNA, RCA/SNA, WGA/SNA, DSL/SNA, GSL-I/SNA, AAL/SNA, GNL/MAL-II, ConA/MAL-II, HHL/MAL-II, NPL/MAL-II, LCA/MAL-II, PHA-E/MAL-II, PHA-L/MAL-II, RCA/MAL-II, PHA-E/ConA, PHA-E/GNL, PHA-L/ConA, PHA-L/GNL, WGA/MAL-II, GSL-I/MAL-II, AAL/MAL-II, RCA/GNL, AAL/ConA, AAL/GNL, AAL/HHL, AAL/NPL, GSL-I/ConA, GSL-I/GNL, GSL-I/HHL, GSL-I/NPL, GSL-I/LCA, DSL/ConA, WGA/ConA, WGA/GNL, WGA/HHL, WGA/NPL, WGA/LCA, RCA/ConA, GNL/ConA.

*Step 1:* In the first step the serum sample and the control serum of similar age and sex, eventually mixed serum from healthy individuals without congenital disorder of glycosylation (hereinafter as "the control serum"), shall be appropriately diluted (in the range from 1:100 to 1:1000000), whereas this dilution must be same for all samples measured by using the given lectin.

The serum sample can be denatured by temperature or otherwise - for example by using chemical agents, microwave radiation, or a combination of the two. Denaturation will allow steric (space) opening of serum macromolecules, thus helping better interaction of the exposed epitopes with individual lectins.

*Step 2:* In the second step a microarray biochip or a microplate is prepared by applying samples and physical or chemical immobilization of the analyzed sample and the control serum onto the surface of the microarray substrate (such as modified glass) or into the wells of a sorption microplate. Microarray substrate is called microarray biochip after the samples are applied.

*Step 3:* The prepared microarray biochip or microplate with immobilized samples is then incubated with a panel of biotinylated lectins (the concentration of which ranges within 0.01 and 100 µg/ml). Then it is incubated with fluorescently or spectrophotometrically (for example, peroxidase) labelled streptavidin conjugate, or another non-glycosylated protein, for example, neutravidin.

The panel of lectins is a set of a minimum of two lectins with the same or different dominant glycan specificity. The panel of lectins contains at least two of the following lectins, either as isolated proteins or as recombinant proteins: Aleuria aurantia lectin (AAL), Arachis hypogaea agglutinin (PNA), Concanavalin A (Con A), Datura stramonium lectin (DSL), Galanthus nivalis lectin (GNL), Griffonia simplicifolia lectin-I (GSL-I), Griffonia simplicifolia lectin-II (GSL-II), Hippeastrum hybrid lectin (HHL), Lens culinaris agglutinin (LCA), Lotus tetragonolobus lectin (LTL), Maackia amurensis lectin-I (MAL-I), Maackia amurensis lectin-II (MAL-II), Narcissus pseudonarcissus (daffodil) lectin (NPL), Phaseolus vulgaris erythtroagglutinin (PHA-E), Phaseolus vulgaris leukoagglutinin (PHA-L), Ricinus communis agglutinin-I (RCA), Sambucus nigra agglutinin (SNA), Ulex europaeus agglutinin I (UEA-I), Wheat germ agglutinin (WGA), Aspergillus oryzae lectin (AOL).

Examples of groups of lectins with similar dominant glycan specificity: for fucose (Fuc); for galactose (Gal); for sialic acid (Sia); for mannose (Man); for N-acetylgalactosamine (GalNAc); for N-acetylglucosamine (GlcNAc). Lectins with similar dominant glycan specificity can differ in their exact glycan specificity. Therefore, two or more lectins with similar dominant glycan specificity can be included in the panel of lectins. Examples of lectins and their dominant glycan specificity can be found in Table No. 1.

**Table No. 1. Examples of lectins and their dominant glycan specificity**

| **Lectin** | **Denomination** | **Dominant glycan specificity** |
|---|---|---|
| Aleuria aurantia lectin | AAL | Fucose (Fuc) |
| Lotus tetragonolobus lectin | LTL | Fucose (Fuc) |
| Ulex europaeus agglutinin I | UEA-I | Fucose (Fuc) |
| Aspergillus oryzae lectin | AOL | Fucose (Fuc) |
| Arachis hypogaea agglutinin | PNA | Galactose (Gal) |
| Maackia amurensis lectin-I | MAL-I | Galactose (Gal) |
| Phaseolus vulgaris erythtroagglutinin | PHA-E | Galactose (Gal) |
| Phaseolus vulgaris leukoagglutinin | PHA-L | Galactose (Gal) |
| Ricinus communis agglutinin-I | RCA | Galactose (Gal) |
| Maackia amurensis lectin-II | MAL-II | Sialic acid (Sia) |
| Sambucus nigra agglutinin | SNA | Sialic acid (Sia) |
| Concanavalin A | ConA | Mannose (Man) |
| Galanthus nivalis lectin | GNL | Mannose (Man) |
| Hippeastrum hybrid lectin | HHL | Mannose (Man) |
| Lens culinaris agglutinin | LCA | Mannose (Man) |
| Narcissus pseudonarcissus lectin | NPL | Mannose (Man) |
| Griffonia simplicifolia lectin-I | GSL-I | N-acetylgalactosamine (GalNAc) |
| Datura stramonium lectin | DSL | N-acetylglucosamine (GlcNAc) |
| Griffonia simplicifolia lectin-II | GSL-II | N-acetylglucosamine (GlcNAc) |
| Wheat germ agglutinin | WGA | N-acetylglucosamine (GlcNAc) |

*Step 4:* In the case of using a fluorescent label, the signal detection is carried out by measuring fluorescence intensity, and in the case of using peroxidase labelling, after adding the appropriate chromogenic substrate, spectrophotometrically, always using the appropriate reading device. In the case of a microarray biochip, it is the microarray scanner and in the case of a microplate with samples, it is a microplate reader.

*Step 5:* Determining the presence of a congenital disorder of glycosylation, or its possible subtype, by calculating the value of the ratio of the interaction signals of control serum with a pair of lectins from the panel, and by proportioning this value with the value of the ratio of the interaction signals of the examined sample (human serum) with the same pair of lectins from the panel. In case the resulting ratio substantially differentiates from a value of 1 (is lesser than 0.5 or greater than 2), the examined sample shall be evaluated as positive for the possible presence of a congenital disorder of glycosylation.

Examples of ratios of pairs of lectins to calculate the value of the ratio of the sample interaction signals with these lectins:
GNL/SNA, ConA/SNA, HHL/SNA, NPL/SNA, LCA/SNA, PHA-E/SNA, PHA-L/SNA, RCA/SNA, WGA/SNA, DSL/SNA, PNA/SNA, GSL-I/SNA, GSL-II/SNA, AAL/SNA, LTL/SNA, UEA-I/SNA, GNL/MAL-I, ConA/MAL-I, HHL/MAL-I, NPL/MAL-I, LCA/MAL-I, PHA-E/MAL-I, PHA-L/MAL-I, RCA/MAL-I, AOL/MAL-I, WGA/MAL-I, DSL/MAL-I, PNA/MAL-I, GSL-I/MAL-I, GSL-II/MAL-I, AAL/MAL-I, LTL/MAL-I, UEA-I/MAL-I, GNL/MAL-II, ConA/MAL-II, HHL/MAL-II, NPL/MAL-II, LCA/MAL-II, PHA-E/MAL-II, PHA-L/MAL-II, RCA/MAL-II, AOL/MAL-II, PHA-E/ConA, PHA-E/GNL, PHA-L/ConA, PHA-L/GNL, WGA/MAL-II, DSL/MAL-II, PNA/MAL-II, GSL-I/MAL-II, GSL-II/MAL-II, AAL/MAL-II, LTL/MAL-II, UEA-I/MAL-II, AOL/ConA, AOL/SNA, AOL/GNL, PNA/GNL, LTL/GNL, UEA-I/GNL, RCA/GNL, AAL/ConA, AAL/GNL, AAL/HHL, AAL/NPL, AOL/HHL, AOL/NPL, GSL-I/ConA, GSL-I/GNL, GSL-I/HHL, GSL-I/NPL, GSL-I/LCA, DSL/ConA, DSL/GNL, DSL/HHL, DSL/NPL, DSL/LCA, GSL-II/ConA, GSL-II/GNL, GSL-II/HHL, GSL-II/NPL, GSL-II/LCA, WGA/ConA, WGA/GNL, WGA/HHL, WGA/NPL, WGA/LCA, LTL/ConA, UEA-I/ConA, PNA/ConA, RCA/ConA, GNL/ConA.

The order of using the lectins in the pair is irrelevant. The value of the ratio lesser than 0.5 or greater than 2 is the parameter. That means if the value of the ratio is let us say greater than 2, then should the lectins' order in the pair be changed it will be less than 0.5.

### Examples

### Example 1

The samples of the analyzed and of the control serum in a volume of 1 µl shall be diluted with physiological phosphate-buffered saline (PBS) in the ratio of 1:1000 and will be denatured with the aim of sterically releasing glyco-epitopes into space, while the covalent bonds are preserved. Denaturation shall be carried out by adding 0.1% SDS and subsequent heating up to the temperature of 100°C during a 10-minute period. A microrobotic device for the application of samples in the microarray format will apply the diluted serum sample and control sample onto the microarray substrate with an epoxy-modified surface that is able to bind proteins, onto 8 fields, triplicate in each field. Thus the sample shall be chemically immobilized on the surface of the microarray substrate. The volume of the sample applied in one place is up to 5 nL. After 12 hours, a mask with 8 fields is placed on the prepared microarray biochip and blocked with a 3% albumin solution. Then the microarray biochip shall be rinsed and subsequently incubated for 1 hour with a panel of biotinylated lectins SNA, MAL-II, ConA, PHA-E, PHA-L, WGA, RCA, GSL-I, GNL, LCA, HHL with the concentration of 25 µg/ml, whereas the respective pairs of lectins used for the calculation of signal ratios for the detection of possible disorders of glycosylation are stated in Table No. 2. After incubation with the panel of lectins, the microarray biochip shall be rinsed and will be incubated for 15 minutes with a fluorescent label conjugated with streptavidin with the concentration of 0.5 µg/ml. After incubation with the streptavidin conjugate - fluorescent label the microarray biochip shall be rinsed again, it will be dried and the level of the fluorescent signal will be measured using the microarray scanner (the resulting value is the average of the triplicate). Ratios of signals for the selected pair of lectins shall be calculated individually for the serum sample and for the control sample (ratios No. 1). Then the ratio of these calculated ratios of signals (ratios No. 1) shall be calculated for the selected pair of lectins and the ratio No. 2 shall be achieved. Based on the value of ratios No. 2 determined for several selected pairs of lectins the possible presence of a congenital disorder of glycosylation shall be stated, eventually, the possible subtype can be proposed, too. Lectins in the panel work synergistically, i. e. the highest informative value of the analytical method is achieved by their specifically selected combination. In case the ratio No. 2 for the analyzed sample and the given lectin are significantly different from number 1 (lesser than 0.5 or greater than 2), the analyzed sample is evaluated as positive for the possible presence of congenital disorder of glycosylation. The measurement results for the determined serum sample are given in Table No. 2.

**Table No. 2**

| **Measured lectins** | **Ratio No. 2** |
|---|---|
| SNA/ConA | 1.795 |
| SNA/PHA-E | 1.411 |
| SNA/WGA | 0.981 |
| SNA/PHA-L | 1.120 |
| SNA/RCA | 0.758 |
| SNA/GNL | **9.982** |
| SNA/GSL-I | 1.770 |
| SNA/HHL | **4.397** |
| SNA/LCA | 1.895 |
| MAL-II/GNL | **5.396** |
| MAL-II/HHL | **2.377** |
| ConA/MAL-II | 1.030 |
| NPL/MAL-II | 0.562 |
| LCA/MAL-II | 0.976 |
| PHA-E/MAL-II | 1.311 |
| PHA-L/MAL-II | 1.652 |
| RCA/MAL-II | **2.441** |
| PHA-E/ConA | 1.272 |
| PHA-E/GNL | **7.073** |
| PHA-L/ConA | 1.603 |
| PHA-L/GNL | **8.914** |
| WGA/MAL-II | 1.886 |
| GSL-I/MAL-II | 1.045 |
| AAL/MAL-II | 1.135 |
| RCA/GNL | **13.170** |
| AAL/HHL | **2.698** |
| GSL-I/ConA | 1.014 |
| GSL-I/GNL | **5.639** |
| GSL-I/HHL | **2.484** |
| GSL-I/NPL | 1.861 |
| GSL-I/LCA | 1.071 |
| WGA/ConA | 1.830 |
| WGA/GNL | **10.178** |
| WGA/HHL | **4.484** |
| WGA/NPL | **3.358** |
| WGA/LCA | 1.932 |
| RCA/ConA | **2.368** |

Based on ratios No. 2 for the measured pairs of lectins SNA/GNL, SNA/HHL, MAL-II/GNL, MAL-II/HHL, RCA/MAL-II, PHA-E/GNL, PHA-L/GNL, RCA/GNL, AAL/HHL, GSL-I/GNL, GSL-I/HHL, WGA/GNL, WGA/HHL, WGA/NPL and RCA/ConA (the ratio No. 2 is lesser than 0.5 or greater than 2), it can be estimated from Table No. 2 that in case of the analyzed serum sample the presence of congenital disorder of glycosylation is possible.

### Example 2

The samples of the analyzed and of the control serum in a volume of 1 µl shall be diluted with physiological phosphate-buffered saline (PBS) in the ratio of 1:1000. A microrobotic device for the application of samples in the microarray format will apply the diluted serum sample and control sample onto the microarray substrate with an epoxy-modified surface that is able to bind proteins, onto 8 fields, triplicate in each field. This way the sample will be chemically immobilized on the surface of the microarray substrate. The further procedure is similar to Example 1. The measurement results for the serum sample that is to be determined are stated in Table No. 3.

**Table No. 3**

| **Measured lectins** | **Ratio No. 2** |
|---|---|
| SNA/ConA | 1.591 |
| SNA/PHA-E | 1.570 |
| SNA/WGA | 1.073 |
| SNA/PHA-L | 1.214 |
| SNA/RCA | 0.768 |
| SNA/GNL | **7.706** |
| SNA/GSL-I | 1.493 |
| SNA/HHL | **4.207** |
| SNA/LCA | 1.961 |
| MAL-II/GNL | **3.376** |
| MAL-II/HHL | 1.843 |
| ConA/MAL-II | 1.4343 |
| NPL/MAL-II | 0.5843 |
| LCA/MAL-II | 1.1408 |
| PHA-E/MAL-II | 1.4537 |
| PHA-L/MAL-II | 1.846 |
| RCA/MAL-II | **2.9737** |
| PHA-E/ConA | 1.0135 |
| PHA-E/GNL | **4.9077** |
| PHA-L/ConA | 1.2871 |
| PHA-L/GNL | **6.2324** |
| WGA/MAL-II | 1.8267 |
| GSL-I/MAL-II | 1.2315 |
| AAL/MAL-II | 1.4272 |
| RCA/GNL | **10.04** |
| AAL/HHL | **2.6307** |
| GSL-I/ConA | 0.8586 |
| GSL-I/GNL | **4.1578** |
| GSL-I/HHL | **2.2701** |
| GSL-I/NPL | 1.332 |
| GSL-I/LCA | 1.0796 |
| WGA/ConA | 1.4828 |
| WGA/GNL | **7.1801** |
| WGA/HHL | **3.9203** |
| WGA/NPL | **4,4809** |
| WGA/LCA | 1.8643 |
| RCA/ConA | **2.0733** |

Based on ratios No. 2 for the measured pairs of lectins SNA/GNL, SNA/HHL, MAL-II/GNL, RCA/MAL-II, PHA-E/GNL, PHA-L/GNL, RCA/GNL, AAL/HHL, GSL-I/GNL, GSL-I/HHL, WGA/GNL, WGA/HHL, WGA/NPL and RCA/ConA (the ratio No. 2 is lesser than 0.5 or greater than 2), it can be estimated from Table No. 3 that in case of the analyzed serum sample the presence of congenital disorder of glycosylation is possible.

### Example 3

The samples of the analyzed and of the control serum in a volume of 1 µl shall be diluted with physiological phosphate-buffered saline (PBS) in the ratio of 1:1000. Further, the samples shall be diluted to the resulting ratios of 1:10000, 1:25000, 1:50000, 1:100000 and 1:250000. With the help of a micropipette the diluted sera in the volume of 100 µl each shall be applied into the individual wells of a sorption microplate that can bind proteins. Thus, the sample shall be physically immobilized on the surface of the sorption microplate. The prepared microplate with samples shall be blocked with 1% albumin solution after 12 hours. Then the microplate with samples shall be rinsed and incubated for 1 hour with the panel of biotinylated lectins GNL (1 µg/ml), NPL (0,1 µg/ml), DSL (0,1 µg/ml), AAL (0,1 µg/ml), ConA (0,1 µg/ml) a SNA (0,1 µg/ml) for detection of possible glycosylation disorders, whereas the sample diluted at 1:10000 is incubated with the GNL lectin, the sample diluted at 1:25000 is incubated with the NPL lectin, the sample diluted at 1:50000 is incubated with the DSL lectin, the sample diluted at 1:100000 is incubated with the AAL lectin, and the sample diluted at 1:250000 is incubated with the ConA and SNA lectins. Following the incubation with the panel of lectins, the microplate shall be rinsed and shall be incubated with 100 µl of peroxidase enzyme conjugated with streptavidin, dilution 25x, for 30 minutes. After the incubation with the peroxidase enzyme conjugated with streptavidin and rinsing, 100 µl of undiluted TMB substrate shall be added and 10 minutes later 100 µl of 1M H₂SO₄ shall be added to stop the colour reaction. Then signals such as absorbance are measured spectrophotometrically at 450 nm. Ratios of signals for the selected pair of lectins shall be calculated individually for the serum sample and for the control sample (ratios No. 1). Then the ratio of these calculated ratios of signals (ratios No. 1) shall be calculated for the selected pair of lectins and the ratio No. 2 shall be achieved. Based on the value of ratios No. 2 determined for several selected pairs of lectins the possible presence of a congenital disorder of glycosylation shall be stated, eventually, the possible subtype can be proposed as well. Lectins in the panel work synergistically, i. e. the highest informative value of the analytical method is achieved by their specifically selected combination. In case the ratio No. 2 for the examined sample and the given lectin differs significantly from number 1 (is lesser than 0.5 or greater than 2), the examined sample is evaluated as positive for the possible presence of the congenital disorder of glycosylation. The measurement results for the serum sample that is to be determined are stated in Table No. 4.

**Table No. 4**

| **Measured lectins** | **Ratio No. 2** |
|---|---|
| ConA/SNA | 0.831 |
| SNA/GNL | 1.274 |
| DSL/SNA | 1.132 |
| AAL/SNA | 0.524 |
| NPL/SNA | **0.327** |
| AAL/ConA | 0.630 |
| AAL/GNL | 0.667 |
| AAL/NPL | 1.602 |
| DSL/ConA | 1.362 |
| GNL/ConA | 0.944 |

On the basis of this measurement of the non-denatured sera based on ratios No. 2 for the measured pairs of lectins NPL/SNA it can be estimated from Table No. 4 that in the case of the analyzed serum sample the presence of congenital disorder of glycosylation is possible.

### Example 4

The samples of the analyzed and of the control serum in a volume of 1 µl shall be diluted with physiological phosphate-buffered saline (PBS) in the ratio of 1:1000 and denatured with the aim to sterically release the glyco-epitopes into space, while the covalent bond remain preserved. Denaturation shall be carried out by adding 0.1% SDS. Then the samples shall be diluted to the resulting ratios of 1:10000, 1:25000, 1:50000, 1:100000 and 1:250000. With the help of a micropipette the diluted sera in the volume of 100 µl each shall be applied into the individual wells of a sorption microplate that can bind proteins. Thus, the sample shall be physically immobilized on the surface of the sorption microplate. Further on the procedure is similar to Example 3. The measurement results for the serum sample that is to be determined are stated in Table No. 5.

**Table No. 5**

| **Measured lectins** | **Ratio No. 2** |
|---|---|
| ConA/SNA | 0.601 |
| SNA/GNL | **3.014** |
| DSL/SNA | 0.785 |
| AAL/SNA | **0.383** |
| NPL/SNA | **0.380** |
| AAL/ConA | 0.636 |
| AAL/GNL | 1.154 |
| AAL/NPL | 1.006 |
| DSL/ConA | 1.305 |
| GNL/ConA | 0.552 |

Based on ratios No. 2 for the measured pairs of lectins SNA/GNL, AAL/SNA and NPL/SNA (the ratio No. 2 is lesser than 0.5 or greater than 2), it can be estimated from Table No. 5 that in case of the analyzed serum sample the presence of congenital disorder of glycosylation is possible.

### Example 5

The samples of the analyzed and of the control serum in a volume of 1 µl shall be diluted with physiological phosphate-buffered saline (PBS) in the ratio of 1:1000 and denatured with the aim to sterically release the glyco-epitopes into space, while the covalent bond remain preserved. Denaturation shall be carried out by heating up to the temperature of 100°C during a 10-minute period. Then the samples shall be diluted to the resulting ratios of 1:10000, 1:25000, 1:50000, 1:100000 and 1:250000. With the help of a micropipette the diluted sera in the volume of 100 µl each shall be applied into the individual wells of a sorption microplate that can bind proteins. Thus, the sample shall be physically immobilized on the surface of the sorption microplate. Further on the procedure is similar to Example 3. The measurement results for the serum sample that is to be determined are stated in Table No. 6.

**Table No. 6**

| **Measured lectins** | **Ratio No. 2** |
|---|---|
| ConA/SNA | 0.684 |
| SNA/GNL | **2.858** |
| DSL/SNA | 0.822 |
| AAL/SNA | **0.432** |
| NPL/SNA | **0.357** |

Based on ratios No. 2 for the measured pairs of lectins SNA/GNL, AAL/SNA and NPL/SNA (the ratio No. 2 is lesser than 0.5 or greater than 2), it can be estimated from Table No. 6 that in case of the analyzed serum sample the presence of congenital disorder of glycosylation is possible.

### Example 6

The samples of the analyzed and of the control serum in a volume of 1 µl shall be diluted with physiological phosphate-buffered saline (PBS) in the ratio of 1:1000 and denatured with the aim to sterically release the glyco-epitopes into space, while the covalent bond remain preserved. Denaturation shall be carried out using microwave waves for the period of 20 seconds. Then the samples shall be diluted to the resulting ratios of 1:10000, 1:25000, 1:50000, 1:100000 and 1:250000. With the help of a micropipette the diluted sera in the volume of 100 µl each shall be applied into the individual wells of a sorption microplate that can bind proteins. Thus, the sample shall be physically immobilized on the surface of the sorption microplate. Further on the procedure is similar to Example 3. The measurement results for the serum sample that is to be determined are stated in Table No. 7.

**Table No. 7**

| **Measured lectins** | **Ratio No. 2** |
|---|---|
| ConA/SNA | 0.632 |
| SNA/GNL | **2.342** |
| DSL/SNA | 1.059 |
| AAL/SNA | **0.456** |
| NPL/SNA | **0.336** |

Based on ratios No. 2 for the measured pairs of lectins SNA/GNL, AAL/SNA and NPL/SNA (the ratio No. 2 is lesser than 0.5 or greater than 2), it can be estimated from Table No. 7 that in case of the analyzed serum sample the presence of congenital disorder of glycosylation is possible.

### Industrial applicability

The newly developed method utilising the microarray biochip or microplate (detection platform) with an application of lectins serves as a sensitive, simple and quick analytical method for efficient determination of differences in glycosylation of sera, eventually of other samples that contain glycans (clinical, biotechnological, biopharmaceutical) and for other diseases manifesting in change of glycosylation, too.

## Claims

1. A method of detecting congenital disorder of glycosylation (CDG) by using a panel of lectins comprising the following successive steps:
a) Dilution of a sample of analyzed human blood serum or blood plasma as well as of a control serum or plasma with physiological solution to a concentration within the range from 1:100 up to 1:1000000;
b) Preparation of a microarray biochip or a microplate through application and physical or chemical immobilisation of the applied analyzed sample and of the control serum or plasma onto a surface of the microarray substrate or into wells of the microplate;
c) Incubation of the prepared microarray biochip or the microplate with the samples with the panel of biotinylated lectins the concentration of which ranges from 0.01 up to 100 µg/ml and the subsequent incubation with a fluorescently, or spectrophotometrically, labelled conjugate of streptavidin or another non-glycosylated protein; where the panel of lectins is a set of minimum two lectins with the same or different dominant glycan specificity and where the panel of lectins contains lectins in the form of isolated proteins or recombinant proteins: Aleuria aurantia lectin (AAL), Arachis hypogaea agglutinin (PNA), Concanavalin A (Con A), Datura stramonium lectin (DSL), Galanthus nivalis lectin (GNL), Griffonia simplicifolia lectin-I (GSL-I), Griffonia simplicifolia lectin-II (GSL-II), Hippeastrum hybrid lectin (HHL), Lens culinaris agglutinin (LCA), Lotus tetragonolobus lectin (LTL), Maackia amurensis lectin-I (MAL-I), Maackia amurensis lectin-II (MAL-II), Narcissus pseudonarcissus (daffodil) lectin (NPL), Phaseolus vulgaris erythtroagglutinin (PHA-E), Phaseolus vulgaris leukoagglutinin (PHA-L), Ricinus communis agglutinin-I (RCA), Sambucus nigra agglutinin (SNA), Ulex europaeus agglutinin I (UEA-I), Wheat germ agglutinin (WGA), Aspergillus oryzae lectin (AOL), where there are lectins with a dominant glycan specificity for fucose (Fuc), for galactose (Gal), for sialic acid (Sia), for mannose (Man), for N-acetylgalactosamine (GalNAc), or for N-acetylglucosamine (GlcNAc);
d) Detection of a signal is carried out, in case of labelling with a fluorescent label, through measurement of fluorescence intensity, and in case of labelling with a peroxidase label spectrophotometrically, always with the appropriate reading device;
e) Determining the presence of a congenital disorder of glycosylation, or its possible subtype, by calculating a value of the ratio of the interaction signals of control serum, or plasma, with a pair of lectins from the panel, and by proportioning this value with the value of the ratio of the interaction signals of the examined sample with the same pair of lectins from the panel; where in case that the resulting ratio substantially differentiates from value of 1, is lesser than 0.5 or greater than 2, the examined sample shall be evaluated as positive for the possible presence of a congenital disorder of glycosylation, while the order in which the lectins in the pair were used does not matter.

2. The method of detecting CDG according to the claim 1, **characterized in that** in the step a) the diluted human serum or plasma as well as the control serum or plasma is denatured using chemical agents and/or by temperature and/or by microwave radiation, or their combination.

3. The method of detecting CDG according to the claim 1 or 2, **characterized in that** a carrier is the microarray substrate or the sorption microplate.

4. The method of detecting CDG according to the claim 1, 2 or 3, **characterized in that** the pairs of lectins for calculation of the value of the sample signal interaction ratio with these lectins are GNL/SNA, ConA/SNA, HHL/SNA, NPL/SNA, LCA/SNA, PHA-E/SNA, PHA-L/SNA, RCA/SNA, WGA/SNA, DSL/SNA, PNA/SNA, GSL-I/SNA, GSL-II/SNA, AAL/SNA, LTL/SNA, UEA-I/SNA, GNL/MAL-I, ConA/MAL-I, HHL/MAL-I, NPL/MAL-I, LCA/MAL-I, PHA-E/MAL-I, PHA-L/MAL-I, RCA/MAL-I, AOL/MAL-I, WGA/MAL-I, DSL/MAL-I, PNA/MAL-I, GSL-I/MAL-I, GSL-II/MAL-I, AAL/MAL-I, LTL/MAL-I, UEA-I/MAL-I, GNL/MAL-II, ConA/MAL-II, HHL/MAL-II, NPL/MAL-II, LCA/MAL-II, PHA-E/MAL-II, PHA-L/MAL-II, RCA/MAL-II, AOL/MAL-II, PHA-E/ConA, PHA-E/GNL, PHA-L/ConA, PHA-L/GNL, WGA/MAL-II, DSL/MAL-II, PNA/MAL-II, GSL-I/MAL-II, GSL-II/MAL-II, AAL/MAL-II, LTL/MAL-II, UEA-I/MAL-II, AOL/ConA, AOL/SNA, AOL/GNL, PNA/GNL, LTL/GNL, UEA-I/GNL, RCA/GNL, AAL/ConA, AAL/GNL, AAL/HHL, AAL/NPL, AOL/HHL, AOL/NPL, GSL-I/ConA, GSL-I/GNL, GSL-I/HHL, GSL-I/NPL, GSL-I/LCA, DSL/ConA, DSL/GNL, DSL/HHL, DSL/NPL, DSL/LCA, GSL-II/ConA, GSL-II/GNL, GSL-II/HHL, GSL-II/NPL, GSL-II/LCA, WGA/ConA, WGA/GNL, WGA/HHL, WGA/NPL, WGA/LCA, LTL/ConA, UEA-I/ConA, PNA/ConA, RCA/ConA, GNL/ConA.

5. The method of detecting CDG according to the claim 4, **characterized in that** the pairs of lectins for calculation of the value of the sample signal interaction ratio with these lectins are GNL/SNA, ConA/SNA, HHL/SNA, NPL/SNA, LCA/SNA, PHA-E/SNA, PHA-L/SNA, RCA/SNA, WGA/SNA, DSL/SNA, GSL-I/SNA, AAL/SNA, GNL/MAL-II, ConA/MAL-II, HHL/MAL-II, NPL/MAL-II, LCA/MAL-II, PHA-E/MAL-II, PHA-L/MAL-II, RCA/MAL-II, PHA-E/ConA, PHA-E/GNL, PHA-L/ConA, PHA-L/GNL, WGA/MAL-II, GSL-I/MAL-II, AAL/MAL-II, RCA/GNL, AAL/ConA, AAL/GNL, AAL/HHL, AAL/NPL, GSL-I/ConA, GSL-I/GNL, GSL-I/HHL, GSL-I/NPL, GSL-I/LCA, DSL/ConA, WGA/ConA, WGA/GNL, WGA/HHL, WGA/NPL, WGA/LCA, RCA/ConA, GNL/ConA.

## Patentansprüche

1. A Verfahren zum Nachweis einer angeborenen Glykosylierungsstörung (CDG) mittels eines Lektinpanels, das die folgenden aufeinanderfolgenden Schritte umfasst:
a) Verdünnung einer Probe des zu analysierenden menschlichen Blutserums oder Blutplasmas sowie eines Kontrollserums oder -plasmas mit physiologischer Kochsalzlösung auf eine Konzentration im Bereich von 1:100 bis 1:1.000.000;
b) Herstellung eines Microarray-Biochips oder einer Mikrotiterplatte durch Aufbringen und physikalische oder chemische Immobilisierung der aufgebrachten zu analysierenden Probe und des Kontrollserums oder -plasmas auf eine Oberfläche des Microarray-Substrats oder in Vertiefungen der Mikrotiterplatte;
c) Inkubation des vorbereiteten Microarray-Biochips oder der Mikrotiterplatte mit den Proben mit dem Panel biotinylierter Lektine, deren Konzentration im Bereich von 0,01 bis 100 µg/ml liegt, und anschließende Inkubation mit einem fluoreszenzmarkierten oder spektrophotometrisch markierten Konjugat aus Streptavidin oder einem anderen nichtglykosylierten Protein; wobei das Panel von Lektinen aus mindestens zwei Lektinen mit gleicher oder unterschiedlicher dominanter Glykanspezifität besteht und wobei das Panel von Lektinen Lektine in Form von isolierten Proteinen oder rekombinanten Proteinen enthält: Aleuria aurantia-Lektin (AAL), Arachis hypogaea-Agglutinin (PNA), Concanavalin A (Con A), Datura stramonium-Lektin (DSL), Galanthus nivalis-Lektin (GNL), Griffonia simplicifolia-Lektin-I (GSL-I), Griffonia simplicifolia-Lektin-II (GSL-II), Hippeastrum-Hybrid-Lektin (HHL), Lens culinaris-Agglutinin (LCA), Lotus tetragonolobus-Lektin (LTL), Maackia amurensis-Lektin-I (MAL-I), Maackia amurensis-Lektin-II (MAL-II), Narcissus pseudonarcissus (Narzisse)-Lektin (NPL), Phaseolus vulgaris-Erythroagglutinin (PHA-E), Phaseolus vulgaris-Leukoagglutinin (PHA-L), Ricinus communis-Agglutinin-I (RCA), Sambucus nigra-Agglutinin (SNA), Ulex europaeus-Agglutinin I (UEA-I), Weizenkeim-Agglutinin (WGA), Aspergillus oryzae-Lektin (AOL), wobei es Lektine mit einer dominanten Glykanspezifität für Fucose (Fuc), für Galactose (Gal), für Sialinsäure (Sia), für Mannose (Man), für N-Acetylgalactosamin (GalNAc) oder für N-Acetylglucosamin (GlcNAc) gibt;
d) Die Signalerkennung erfolgt bei Markierung mit einer Fluoreszenzmarkierung durch Messung der Fluoreszenzintensität und bei Markierung mit einer Peroxidase-Markierung spektrophotometrisch, stets unter Verwendung des entsprechenden Messgeräts;
e) Bestimmung des Vorliegens einer angeborenen Glykosylierungsstörung oder ihres möglichen Subtyps durch Berechnung eines Wertes für das Verhältnis der Interaktionssignale von Kontrollserum oder -plasma mit einem Lektinpaar aus dem Panel und durch Vergleich dieses Wertes mit dem Wert für das Verhältnis der Interaktionssignale der untersuchten Probe mit demselben Lektinpaar aus dem Panel; wobei für den Fall, dass das resultierende Verhältnis wesentlich vom Wert 1 abweicht, kleiner als 0,5 oder größer als 2 ist, die untersuchte Probe als positiv auf das mögliche Vorliegen einer angeborenen Glykosylierungsstörung bewertet wird, während die Reihenfolge, in der die Lektine des Paares verwendet wurden, keine Rolle spielt.

2. Verfahren zum Nachweis von CDG gemäß Anspruch 1, **dadurch gekennzeichnet, dass** in Schritt a) das verdünnte menschliche Serum oder Plasma sowie das Kontrollserum oder - plasma unter Verwendung chemischer Mittel und/oder durch Temperatur und/oder durch Mikrowellenstrahlung oder eine Kombination davon denaturiert wird.

3. Verfahren zum Nachweis von CDG nach Anspruch 1 oder 2, **dadurch gekennzeichnet, dass** ein Träger das Microarray-Substrat oder die Sorptions-Mikrotiterplatte ist.

4. Verfahren zum Nachweis von CDG nach Anspruch 1, 2 oder 3, **dadurch gekennzeichnet, dass** die Lektinpaare zur Berechnung des Wertes des Proben-Signal-Wechselwirkungsverhältnisses mit diesen Lektinen GNL/SNA, ConA/SNA, HHL/SNA, NPL/SNA, LCA/SNA, PHA-E/SNA, PHA-L/SNA, RCA/SNA, WGA/SNA, DSL/SNA, PNA/SNA, GSL-I/SNA, GSL-II/SNA, AAL/SNA, LTL/SNA, UEA-I/SNA, GNL/MAL-I, ConA/MAL-I, HHL/MAL-I, NPL/MAL-I, LCA/MAL-I, PHA-E/MAL-I, PHA-L/MAL-I, RCA/MAL-I, AOL/MAL-I, WGA/MAL-I, DSL/MAL-I, PNA/MAL-I, GSL-I/MAL-I, GSL-II/MAL-I, AAL/MAL-I, LTL/MAL-I, UEA-I/MAL-I, GNL/MAL-II, ConA/MAL-II, HHL/MAL-II, NPL/MAL-II, LCA/MAL-II, PHA-E/MAL-II, PHA-L/MAL-II, RCA/MAL-II, AOL/MAL-II, PHA-E/ConA, PHA-E/GNL, PHA-L/ConA, PHA-L/GNL, WGA/MAL-II, DSL/MAL-II, PNA/MAL-II, GSL-I/MAL-II, GSL-II/MAL-II, AAL/MAL-II, LTL/MAL-II, UEA-I/MAL-II, AOL/ConA, AOL/SNA, AOL/GNL, PNA/GNL, LTL/GNL, UEA-I/GNL, RCA/GNL, AAL/ConA, AAL/GNL, AAL/HHL, AAL/NPL, AOL/HHL, AOL/NPL, GSL-I/ConA, GSL-I/GNL, GSL-I/HHL, GSL-I/NPL, GSL-I/LCA, DSL/ConA, DSL/GNL, DSL/HHL, DSL/NPL, DSL/LCA, GSL-II/ConA, GSL-II/GNL, GSL-II/HHL, GSL-II/NPL, GSL-II/LCA, WGA/ConA, WGA/GNL, WGA/HHL, WGA/NPL, WGA/LCA, LTL/ConA, UEA-I/ConA, PNA/ConA, RCA/ConA, GNL/ConA sind.

5. Verfahren zum Nachweis von CDG gemäß Anspruch 4, **dadurch gekennzeichnet, dass** die Lektinpaare zur Berechnung des Wertes des Proben-Signal-Interaktionsverhältnisses mit diesen Lektinen GNL/SNA, ConA/SNA, HHL/SNA, NPL/SNA, LCA/SNA, PHA-E/SNA, PHA-L/SNA, RCA/SNA, WGA/SNA, DSL/SNA, GSL-I/SNA, AAL/SNA, GNL/MAL-II, ConA/MAL-II, HHL/MAL-II, NPL/MAL-II, LCA/MAL-II, PHA-E/MAL-II, PHA-L/MAL-II, RCA/MAL-II, PHA-E/ConA, PHA-E/GNL, PHA-L/ConA, PHA-L/GNL, WGA/MAL-II, GSL-I/MAL-II, AAL/MAL-II, RCA/GNL, AAL/ConA, AAL/GNL, AAL/HHL, AAL/NPL, GSL-I/ConA, GSL-I/GNL, GSL-I/HHL, GSL-I/NPL, GSL-I/LCA, DSL/ConA, WGA/ConA, WGA/GNL, WGA/HHL, WGA/NPL, WGA/LCA, RCA/ConA, GNL/ConA sind.

## Revendications

1. Procédé de détection des troubles congénitaux de la glycosylation (CDG) à l'aide d'un panel de lectines, comprenant les étapes successives suivantes :
a) Dilution d'un échantillon de sérum ou de plasma sanguin humain à analyser ainsi que d'un sérum ou d'un plasma témoin dilué dans une solution physiologique à une concentration comprise entre 1 : 100 et 1 : 1 000 000 ;
b) Préparation d'une puce à microarrays ou d'une microplaque par application et immobilisation physique ou chimique de l'échantillon analysé utilisé et du sérum ou plasma témoin sur une surface du substrat de la biopuce ou dans les puits de la microplaque ;
c) Incubation de la biopuce à microarrays préparée ou de la microplaque avec les échantillons avec le panel de lectines biotinylées dont la concentration varie de 0,01 à 100 µg/ml et l'incubation subséquente avec un conjugué de streptavidine ou d'une autre protéine non glycosylée, marqué par fluorescence ou par spectrophotométrie ; où le panel de lectines est composé d'au moins deux lectines présentant une spécificité glycanique dominante identique ou différente et où le panel de lectines comprend des lectines sous forme de protéines isolées ou de protéines recombinantes : lectine d'Aleuria aurantia (AAL), agglutinine d'Arachis hypogaea (PNA), Concanavaline A (Con A), lectine de Datura stramonium (DSL), lectine de Galanthus nivalis (GNL), lectine-I de Griffonia simplicifolia (GSL-I), lectine-II de Griffonia simplicifolia (GSL-II), lectine hybride d'Hippeastrum (HHL), agglutinine de Lens culinaris (LCA), lectine de Lotus tetragonolobus (LTL), lectine-I de Maackia amurensis (MAL-I), lectine-II de Maackia amurensis (MAL-II), lectine de Narcissus pseudonarcissus (jonquille) (NPL), érythroagglutinine de Phaseolus vulgaris (PHA-E), leucoagglutinine de Phaseolus vulgaris (PHA-L), agglutinine-I de Ricinus communis (RCA), agglutinine de Sambucus nigra (SNA), agglutinine-I de Ulex europaeus (UEA-I), agglutinine de germe de blé (WGA), lectine d'Aspergillus oryzae (AOL), où se trouvent des lectines présentant une spécificité glycanique dominante pour le fucose (Fuc), pour le galactose (Gal), pour l'acide sialique (Sia), pour le mannose (Man), pour la N-acétylgalactosamine (GalNAc) ou pour la N-acétylglucosamine (GlcNAc) ;
d) en cas de marquage par un marqueur fluorescent, la détection du signal s'effectue par la mesure de l'intensité de la fluorescence ; en cas de marquage spectrophotométrique à l'aide d'un marqueur à peroxydase, la détection du signal s'effectue toujours à l'aide d'un appareil de lecture adéquat ;
e) détermination des troubles congénitaux de la glycosylation, ou ses sous-types éventuels, en calculant la valeur du rapport entre les signaux d'interaction du sérum ou du plasma témoin avec une paire de lectines du panel et en proportionnant cette valeur à celle du rapport entre les signaux d'interaction de l'échantillon examiné et ceux obtenus avec la même paire de lectines du panel ; où, si le rapport obtenu s'écarte sensiblement de la valeur 1, s'il est inférieur à 0,5 ou supérieur à 2, l'échantillon examiné sera considéré comme positif quant à la présence éventuelle d'un trouble congénital de la glycosylation, alors que l'ordre dans lequel les lectines de la paire sont utilisées ne joue aucun rôle.

2. Procédé de détection des CDG selon la revendication 1, **caractérisé en ce que** dans l'étape a) le sérum ou le plasma humain dilué ainsi que le sérum ou le plasma témoin sont dénaturés à l'aide d'agents chimiques et/ou par la température et/ou par rayonnement micro-ondes, ou combinaison de ceux-ci.

3. Procédé de détection des CDG selon la revendication 1 ou 2, **caractérisé en ce que** le support est le substrat de la biopuce à microarrays ou la microplaque de sorption.

4. Procédé de détection des CDG selon la revendication 1, 2 ou 3, **caractérisé en ce que** les paires de lectines pour le calcul de la valeur du rapport d'interaction du signal de l'échantillon avec ces lectines sont GNL/SNA, ConA/SNA, HHL/SNA, NPL/SNA, LCA/SNA, PHA-E/SNA, PHA-L/SNA, RCA/SNA, WGA/SNA, DSL/SNA, PNA/SNA, GSL-I/SNA, GSL-II/SNA, AAL/SNA, LTL/SNA, UEA-I/SNA, GNL/MAL-I, ConA/MAL-I, HHL/MAL-I, NPL/MAL-I, LCA/MAL-I, PHA-E/MAL-I, PHA-L/MAL-I, RCA/MAL-I, AOL/MAL-I, WGA/MAL-I, DSL/MAL-I, PNA/MAL-I, GSL-I/MAL-I, GSL-II/MAL-I, AAL/MAL-I, LTL/MAL-I, UEA-I/MAL-I, GNL/MAL-II, ConA/MAL-II, HHL/MAL-II, NPL/MAL-II, LCA/MAL-II, PHA-E/MAL-II, PHA-L/MAL-II, RCA/MAL-II, AOL/MAL-II, PHA-E/ConA, PHA-E/GNL, PHA-L/ConA, PHA-L/GNL, WGA/MAL-II, DSL/MAL-II, PNA/MAL-II, GSL-I/MAL-II, GSL-II/MAL-II, AAL/MAL-II, LTL/MAL-II, UEA-I/MAL-II, AOL/ConA, AOL/SNA, AOL/GNL, PNA/GNL, LTL/GNL, UEA-I/GNL, RCA/GNL, AAL/ConA, AAL/GNL, AAL/HHL, AAL/NPL, AOL/HHL, AOL/NPL, GSL-I/ConA, GSL-I/GNL, GSL-I/HHL, GSL-I/NPL, GSL-I/LCA, DSL/ConA, DSL/GNL, DSL/HHL, DSL/NPL, DSL/LCA, GSL-II/ConA, GSL-II/GNL, GSL-II/HHL, GSL-II/NPL, GSL-II/LCA, WGA/ConA, WGA/GNL, WGA/HHL, WGA/NPL, WGA/LCA, LTL/ConA, UEA-I/ConA, PNA/ConA, RCA/ConA, GNL/ConA.

5. Procédé de détection des CDG selon la revendication 4, **caractérisé en ce que** les paires de lectines pour le calcul de la valeur du rapport d'interaction du signal de l'échantillon avec ces lectines sont GNL/SNA, ConA/SNA, HHL/SNA, NPL/SNA, LCA/SNA, PHA-E/SNA, PHA-L/SNA, RCA/SNA, WGA/SNA, DSL/SNA, PNA/SNA, GSL-I/SNA, AAL/SNA, GNL/MAL-II, ConA/MAL-II, HHL/MAL-II, NPL/MAL-II, LCA/MAL-II, PHA-E/MAL-II, PHA-L/MAL-II, RCA/MAL-II, PHA-E/ConA, PHA-E/GNL, PHA-L/ConA, PHA-L/GNL, WGA/MAL-II, GSL-I/MAL-II, AAL/MAL-II, RCA/GNL, AAL/ConA, AAL/GNL, AAL/HHL, AAL/NPL, GSL-I/ConA, GSL-I/GNL, GSL-I/HHL, GSL-I/NPL, GSL-I/LCA, DSL/ConA, WGA/ConA, WGA/GNL, WGA/HHL, WGA/NPL, WGA/LCA, RCA/ConA, GNL/ConA.
